# EUROPEAN PATENT SPECIFICATION

(11) **EP 2 056 784 B1**
(45) Date of publication and mention of the grant of the patent: **31.08.2011**
(21) Application number: 07849160.2
(22) Date of filing: 15.11.2007
(51) Int. Cl.: A61K 8/895, A61K 8/58, A61L 15/44

(54) **DIAPER RASH COMPOSITION AND METHOD**
ZUSAMMENSETZUNG GEGEN WINDELBEDINGTEN HAUTAUSSCHLAG UND DESSEN BEHANDLUNG
COMPOSITION POUR ÉRYTHÈME FESSIER ET PROCÉDÉ DE TRAITEMENT CORRESPONDANT

(30) Priority: 15.12.2006 US 640431
(43) Date of publication of application: 13.05.2009
(73) Proprietor: KIMBERLY-CLARK WORLDWIDE, INC., Neenah, WI 54956 (US)
(72) Inventor: LANGOLF, Brian M., Fond Du Lac, Wisconsin 54937 (US); WENZEL, Scott W., Neenah, Wisconsin 54956 (US); CUNNINGHAM, Corey Thomas, Larsen, Wisconsin 54947 (US); FLUGGE-BERENDES, Lisa, Appleton, Wisconsin 54914 (US)
(74) Representative: Zimmermann & Partner
(86) International application number: PCT/IB2007/054670
(87) International publication number: WO 2008/075231

(56) References cited:
- EP-A- 1 637 120
- EP-A1- 0 388 110
- EP-A2- 1 579 845
- WO-A-00/71177
- WO-A-01/85128
- WO-A-98/00105
- WO-A1-2006/041764
- WO-A2-02/03952
- WO-A2-2005/092347
- US-A1- 2005 058 669

## Description

### BACKGROUND

Diaper rash, or diaper dermatitis, is an inflammatory skin condition that can result from a number of different causes. In some cases, the inflammatory skin condition is a direct result of wearing a diaper; however, the rash could have an unrelated origin. In any case, the contact between the rash and the diaper creates an uncomfortable and even painful sensation for the person affected with a diaper rash.

Current diaper rash cream formulations on the market are typically thick, pasty ointment types that are difficult to apply due to their viscous consistency. In addition, because irritated areas of the skin are sore and sensitive to the touch, the application of ointments that are tacky and sticky makes the condition even more unpleasant, uncomfortable, and painful. Furthermore, such products are often messy and difficult to remove from the hands. EP-A-1 637 120 describes aqueous, non-alcoholic liquid powder formulations comprising polymeric emulsifiers and water as a volatile component. WO 01/85128 A is directed at a liquid, water-repellent, substantially anhydrous, spray-pumpable composition. WO 98/00105 describes cosmetic compositions with skin conditioning agents such as exfoliants.

As such, a need currently exists for an improved rash cream formulation that spreads easily on the skin and does not produce a sticky or tacky residue upon application to the skin. Instead, a composition is needed that glides easily on the skin with little or no drag, that leaves a light, silky or powdery feel on the skin, and that leaves the skin feeling perceivably dry.

### SUMMARY

In general, the present disclosure is directed to a diaper rash composition according to claim 1. The diaper rash composition may comprise a volatile silicone, a silicone elastomer, and a diaper rash agent. In accordance with the present disclosure, the diaper rash composition may be a substantially non-aqueous semi-solid, containing no emulsifiers.

In one embodiment, the volatile silicone in the diaper rash composition may comprise cyclomethicone, dimethicone, or mixtures thereof. The volatile silicone may be present in the diaper rash composition in an amount greater than 10% by weight. In another embodiment, the volatile silicone is present in the diaper rash composition in amount greater than 25% by weight.

Another ingredient of the diaper rash composition, the silicone elastomer, may comprise a crosslinked reaction product of a polysiloxane containing a silane bond (≡Si-H group), and an alpha, omega-diene. The polysiloxane containing a ≡Si-H group may comprise R₃SiO(R'₂SiO)ₐ(R"HSiO)_{b}SiR₃, HR₂SiO(R'₂SiO)_{c}SiR₂H, HR₂SiO(R'₂SiO)ₐ(R"HSiO)_{b}SiR₂H, or mixtures thereof, wherein R, R', and R" are alkyl groups with 1 to 6 carbon atoms, a is 0 to 250, b is 1 to 250, and c is 0 to 250. The alpha, omega-diene of the given embodiment comprises CH₂=CH(CH₂)ₓCH=CH₂ wherein x is 1 to 20. In accordance with the present disclosure, the viscosity of the silicone elastomer may be greater than 200 Pa s (200,000 cP). In one embodiment, the silicone elastomer is present in the diaper rash composition in an amount of from 3% to 10% by weight.

A third component of the diaper rash composition may be a diaper rash agent. In accordance with the present disclosure, the diaper rash agent may comprise a zinc oxide or a nonvolatile dimethicone. In one embodiment, the diaper rash agent may be present in the composition in an amount of from 5% to 25% by weight.

In addition to the volatile silicone, the silicone elastomer, and the diaper rash agent present in the diaper rash composition, the composition may optionally contain a non-silicone elastomer, a carrier, a thickener, or combinations thereof. The listed optional ingredients may be completely absent from the composition or may be present in any given combination.

In one embodiment, the diaper rash composition may comprise a non-silicone elastomer which comprises a styrenic block copolymer. In another embodiment, the diaper rash composition may comprise a carrier in an amount of at least 5% by weight. Numerous components may act as carriers in the diaper rash composition. The carrier may comprise, in one particular embodiment, isopropyl myristate, isododecane, or mixtures thereof. The optional thickener may comprise a clay.

The present disclosure is additionally directed to a process for treating a diaper rash comprising applying the diaper rash composition of the present disclosure to a person affected with a diaper rash.

### DETAILED DESCRIPTION

While there are numerous diaper rash creams on the market, many current formulations may not be conducive to effectively alleviating diaper rash irritation. For example, in order to effectively alleviate diaper rash irritation, a diaper rash composition should be comfortable and easy both to apply and to remove. Furthermore, the diaper rash composition should create an effective barrier between the surface of the affected skin area and the diaper. The barrier formed by the diaper rash composition prevents the diaper rash condition from worsening while keeping the person affected with diaper rash comfortable during the recovery process.

The present disclosure, in one embodiment, is generally directed to a diaper rash composition, having both skin protectant and rash cream properties, that spreads easily on the skin and does not produce a sticky or tacky residue upon application to the skin. The diaper rash compositions of the present disclosure are configured to glide on the skin, leaving a light, silky or powdery feel. In comparison, the diaper rash formulations currently available have been found to be pasty, sticky, or tacky. Such qualities hinder easy application and removal of the diaper rash formulation. Furthermore, the current formulations have been found to be unpleasant, uncomfortable, and even painful for the person affected with diaper rash. The light, powdery feel of the diaper rash composition of the present disclosure is generally more pleasant than the feel of the tackier compositions.

The diaper rash composition of the present application may comprise a volatile silicone, a silicone elastomer, and a diaper rash agent. In one embodiment, the given components may be the only components in the composition. In such an embodiment the three listed components combine to form a composition having a light, powdery feel.

The volatile silicone may be present in the diaper rash composition in order to assist in creating a silky and powdery feel. The term "volatile" refers to those materials having a measurable pressure at ambient conditions. In one embodiment, the volatile silicone has a low molecular weight. Volatile polyorganosiloxanes useful herein may be cyclic or linear. Suitable cyclic silicones include polydimethysiloxanes containing from 4 to 8 silicon atoms and an equal number of oxygen atoms in the ring (i.e. D4 to D8). The straight chain silicones that are suitable for the diaper rash composition include those with a | viscosity equal to or less than 5 mm2/s (5 centistokes).

The cyclic silicones suitable for use in the diaper rash composition are generally known as cyclomethicones. In one embodiment, the volatile silicone is cyclomethicone, more specifically cyclopentasiloxane, a compound sold commercially under the name DOW CORNING 245 (distributed by Dow Corning Corp.).

In another embodiment, the diaper rash composition comprises a low viscosity dimethicone, for example, a linear polysiloxane. One example of a commercially available low viscosity dimethicone, for instance, is DOW CORNING 200 dimethicone available from the Dow Corning Corp. DOW CORNING 200 | dimethicone has a viscosity of 5 mm²/s (5 centistoke). Other suitable volatile silicones for use in the diaper rash composition of the present disclosure include, for example, cyclomethicones of varying viscosities, e.g., DOW CORNING 244, DOW CORNING 344, and DOW CORNING 345 (commercially available from Dow Corning Corp.); SF-1204 and SF-1202 Silicone Fluids (commercially available from G.E. Silicones), Silicone 7207 and Silicone 7158 (commercially available from Union Carbide Corp.); and SWS-03314 (commercially available from SWS Silicones Corp.).

The amount of volatile silicone present in the diaper rash composition of the present disclosure depends on a variety of different factors. In one embodiment, the volatile silicone may be present in the diaper rash composition in an amount of greater than 10% by weight. Alternatively, the volatile silicone may be present in the diaper rash composition in amount of greater than 25%. Suitably, the volatile silicone is present in the diaper rash composition in an amount of from 10% to 99% by weight and even more suitably from 25% to 90% by weight.

The second component contained in the diaper rash composition comprises a silicone elastomer. Once the diaper rash composition is applied to the skin, the silicone elastomer, in one embodiment, can form a film on the skin while allowing the volatile silicone to evaporate, leaving a dry feeling on the skin. The diaper rash composition can contain a single silicone elastomer or may contain two or more silicone elastomers.

In one embodiment, the silicone elastomers may be prepared by a crosslinking reaction between (A) ≡Si-H containing polysiloxanes and (B) an alpha, omega-diene in the presence of a platinum catalyst. Typically, commercially available silicone elastomers are sold or supplied as a blend with a silicone (C). The elastomers can be swollen with the low molecular weight polysiloxane under a shear force. Elastomers containing 65-98 weight percent of the low molecular weight polysiloxane are stable and form uniform silicone pastes with a wide viscosity range.

The silicone pastes have excellent properties including clarity, thixotropy, and shear thinning. The materials also spread smoothly on the skin. The silicone elastomers are capable of being crumbled to form a silicone powder. The silicone powder has the unique property of being easily rubbed-in on the skin, and silicone resins can be incorporated therein to improve the substantivity of formulations applied to the skin.

The ≡Si-H containing polysiloxane (A) is represented by compounds of the formula R₃SiO(R'₂SiO)ₐ(R"HSiO)_{b}SiR₃ designated herein as type A¹ and compounds of the formula HR₂SiO(R'₂SᵢO)_{c}SᵢR₂H or formula HR₂SiO(R'₂SiO)ₐ(R"HSiO)_{b}SiR₂H designated herein as type A². In these formulas, R, R', and R", are alkyl groups with 1-6 carbon atoms; a is 0-250; b is 1-250; and c is 0-250. The molar ratio of compounds A²:A¹ is 0-20, preferably 0-5. Compounds of types A¹ and A² may be used in the crosslinking reaction; however, it is possible to successfully conduct the reaction using only compounds of type A¹.

The alpha, omega-diene (B) is a compound of the formula CH₂=CH(CH₂)ₓCH=CH₂ where x is 1-20. Some representative examples of suitable alpha, omega-dienes for use herein are 1,4-pentadiene; 1,5-hexadiene; 1,6-heptadiene; 1,7-octadiene; 1,8-nonadiene; 1,9-decadiene; 1,11-dodecadiene; 1,13-tetradecadiene; and 1,19-eicosadiene.

The addition and crosslinking reaction may require a catalyst to effect the reaction between the ≡Si-H containing polysiloxane and the alpha, omega-diene. Suitable catalysts are Group VIII transition metals, i.e., the noble metals. Such noble metal catalysts are described in U.S. Pat. No. 3,923,705, One preferred platinum catalyst is Karstedt's catalyst, which is described in Karstedt's U.S. Pat. Nos. 3,715,334 and 3,814,730. Karstedt's catalyst is a platinum divinyl tetramethyl disiloxane complex typically containing about one weight percent of platinum in a solvent such as toluene. Another platinum catalyst that may be used is a reaction product of chloroplatinic acid and an organosilicon compound containing terminal aliphatic unsaturation. It is described in U.S. Pat. No. 3,419,593. The noble metal catalysts are used in amounts from 0.00001-0.5 parts per 100 weight parts of the ≡Si-H containing polysiloxane, preferably 0.00001-0.02 parts, most preferably 0.00001-0.002 parts.

The phrase low molecular weight silicone oil (C) is intended to comprise low molecular weight linear and cyclic volatile methyl siloxanes, low molecular weight linear and cyclic volatile and non-volatile alkyl and aryl siloxanes, low molecular weight linear and cyclic functional siloxanes, or combinations thereof.

Silicone elastomers such as those described above are commercially available from multiple sources. For example, silicone elastomers are available through Dow Corning Corporation under the name DOW CORNING 9040 and DOW CORNING 9011. Commercially available silicone elastomer products, such as those identified above, are generally sold as a silicone elastomer blend. In particular, the commercially available products typically contain a silicone elastomer blended with a volatile silicone oil, such as cyclomethicone. More particularly, such blends may have a non-volatile content of less than 20%, such as from 12% to 13%.

The silicone elastomer blends may be present in the diaper rash composition of the present disclosure in amounts that range from 1% to 80% by weight. For instance, the silicone elastomer blends may be present in the composition in an amount from 10% to 70% by weight.

The actual amount of silicone elastomer present in the diaper rash composition, on the other hand, may be from 0.1% to 20% by weight, such as from 1% to 15% by weight, such as from 1% to 10% by weight.

The diaper rash composition of the present disclosure further comprises at least one diaper rash agent, which may be configured to form at least a partial skin barrier. While the diaper rash composition of the present disclosure is intended to glide on easily and be easily applied and removed, the composition can also be configured to form a barrier between the skin and the diaper. The skin barrier shields the skin from the diaper, thus keeping the diaper rash condition from being worsened.

Suitable compounds to serve as diaper rash agents include zinc oxide, a nonvolatile dimethicone, allantoin, cod liver oil, colloidal oatmeal, kaolin, lanolin, petrolatum, topical starch, or combinations thereof.

The amount of the diaper rash agent present in the diaper rash composition may vary greatly depending on the diaper rash agent employed. For example, in one embodiment, allantoin may be present as a diaper rash agent in an amount of from 0.5% to 2% by weight. Diaper rash agents such as dimethicone and zinc oxide may be present in an amount of from 1% to 40% by weight. In one embodiment, cod liver oil, kaolin, lanolin, or a combination thereof may be present in the diaper rash composition in an amount of from 4% to 50%. Petrolatum, colloidal oatmeal, or topical starch may be present in an amount of from 10% to 80% as a diaper rash agent.

The diaper rash composition of the present disclosure can be formulated so as to be substantially non-aqueous. For instance, the diaper rash composition can contain less than 1% by weight water, such as less than 0.5% by weight water. In one particular embodiment, for instance, the diaper rash composition contains no water.

Once formulated, the diaper rash composition is generally a semi-solid. In particular, the composition has some rigidity and has properties intermediate between a solid and a liquid. The composition does not flow and therefore does not have a viscosity as would be measurable for a liquid. The composition, however, can conform to the shape of a container when an adequate force is applied to the composition. The composition can also be spread over a surface, such as one's skin.

Of particular advantage, since the composition is a semi-solid, the composition can be carefully positioned and placed over an infected area without the composition flowing onto non-affected areas.

Another advantage to the diaper rash composition of the present disclosure is that the composition can be formulated without containing any emulsifiers. Many diaper rash compositions produced in the past contain emulsifiers. Emulsifiers, however, not only can add cost to the composition but can also dilute the effectiveness of the diaper rash agent and possibly can interfere with the other ingredients. Some emulsifiers are also known to irritate the skin. Furthermore, emulsifiers increase the hardness of a composition, making the diaper rash composition uncomfortable to the person affected with diaper rash.

In another embodiment, the diaper rash composition comprises at least one of a volatile silicone, a silicone elastomer, a diaper rash agent, and/or a carrier. The carrier may thin the composition, change the feel of the composition, dilute the silicone in the composition, or act in any combination of the above.

Suitable carriers which may be added into the diaper rash composition include the following: light hydrocarbon oil (e.g. mineral oil, isododecane, petrolatum), vegetable or natural oil (e.g. sunflower oil, olive oil, sweet almond oil, grapeseed oil, corn oil, safflower oil, shea butter, coconut oil, canola oil, castor oil, jojoba oil), hydrogenated vegetable oil (e.g. hydrogenated castor wax, hydrogenated apricot kernel oil, hydrogenated canola oil, hydrogenated jojoba oil, hydrogenated olive oil, hydrogenated sesame seed oil), fatty ester (e.g. octyldodecyl neopentanoate, stearyl stearate, isopropyl myristate, isopropyl palmitate, stearyl behenate, C12-C15 alkyl benzoate, butyl isostearate, cetyl caprate, cetyl caprylate, ethyl apricot kernelate, ethyl avocadate, ethylhexyl caprate/caprylate, ethylhexyl cocoate, ethylhexyl isopalmitate, isocetyl myristate, isopropyl jojobate, myristyl laurate), fatty acid (e.g. palmitic acid, stearic acid, myristic acid, oleic acid, linoleic acid, behenic acid), fatty alcohol (e.g. lauryl alcohol, cetyl alcohol, stearyl alcohol, behenyl alcohol), or combinations thereof. In one embodiment, the diaper rash composition may comprise a fatty ester as a carrier. One example of a fatty ester is isopropyl myristate, which is available under the name TEGOSOFT M (Degussa).

If a carrier is present in the diaper rash composition, then the amount may vary greatly depending on a variety of factors. Generally, the carrier is present in an amount greater than 5% by weight. In one embodiment, the carrier is present in an amount of from 5% to 50% by weight. In another embodiment, the carrier is present in an amount of from 5% to 15% by weight.

In one embodiment, when a carrier is present in the diaper rash composition, the diaper rash composition may also optionally contain a non-silicone elastomer. Suitable non-silicone elastomers that may be used in the diaper rash composition include block copolymers, such as styrenic block copolymers. For instance, the block copolymers may have the general formula A-B-A' or A-B, where A and A' are each a thermoplastic polymer end block which contains a styrenic moiety such as a poly(vinyl arene) and where B is an elastomeric polymer midblock such as a conjugated diene or a lower alkene polymer. Block copolymers form the A and A' blocks, and the present block copolymers are intended to embrace linear, branched and radial block copolymers. In this regard, the radial block copolymers may be designated (A-B)ₘ-X, wherein X is a polyfunctional atom or molecule and in which each (A-B)ₘ-radiates from X in a way that A is an endblock. In the radial block copolymer, X may be an organic or inorganic polyfunctional atom or molecule and m is an integer having the same value as the functional group originally present in X. It is usually at least 3, and is frequently 4 or 5, but not limited thereto. Thus, in the present invention, the expression "block copolymer," and particularly "A-B-A" and "A-B" block copolymer, is intended to embrace all block copolymers having such rubbery blocks and thermoplastic blocks as discussed above.

Suitable block copolymers to use in the diaper rash composition include styrene/butadiene/styrene copolymers, styrene/isoprene/styrene copolymers, styrene-ethylene/butylenes-styrene copolymers, styrene-ethylene/propylene-styrene copolymers, (styrene-butadiene) n polymers, (styrene-isoprene) n polymers, styrene-butadiene copolymers, styrene-ethylene/propylene copolymer, and combinations thereof.

In one embodiment, the non-silicone elastomer present in the diaper rash composition may comprise VERSAGEL MD 1600, which is isodecane, a carrier, blended with ethylene/propylene/styrene copolymers and butylene/ethylene/styrene copolymers. VERSAGEL MD 1600 is a non-silicone | elastomer, which has a viscosity of 50 Pa s 50,000 cP) and is commercially available from Penreco. Additional commercial examples of such non-silicone elastomers are, for example, those known as KRATON materials which are available from Shell Chemical Company of Houston, Tex. KRATON block copolymers are available in several different formulations, a number of which are identified in U.S. Pat. Nos. 4,663,220; 4,323,534; 4,834,738; 5,093,422 and 5,304,599.

Polymers composed of an elastomeric A-B-A-B tetrablock copolymer may also be used in the practice of this invention. Such polymers are discussed in U.S. Pat. No. 5,332,613 to Taylor et al. In such polymers, A is a thermoplastic polymer block and B is an isoprene monomer unit hydrogenated to substantially a poly(ethylene-propylene) monomer unit. An example of such a tetrablock copolymer is a styrene-poly(ethylene-propylene)-styrene-poly(ethylene-propylene) or SEPSEP elastomeric block copolymer available from the Shell Chemical Company of Houston, Tex. under the trade designation KRATON G-1657.

Generally, if a non-silicone elastomer is present in the diaper rash composition, then the non-silicone elastomer is present in an amount of from about 0.1 % to about 5% by weight. In one embodiment, the non-silicone elastomer is present in the diaper rash composition in an amount of from about 0.5% to about 3% by weight.

In addition to those listed above, the diaper rash composition may comprise a thickener as another optional component. Suitable thickeners for the diaper rash composition include clay, synthetic clay, silica, a hydrophobically modified cellulosic, kentonite, bentonite, fumed silica, hydroxypropyl methylcellulose, a modified starch, or combinations thereof. In one embodiment, a layered silicate available under the name LAPONITE available from Rockwood Additives Ltd. Corp., is present in the diaper rash composition as a thickener.

If present, a thickener may be included in the composition in an amount of from 0.01% to 10% by weight. In one embodiment, the thickener is present in the diaper rash composition in an amount of from 0.1 % to 5% by weight. In another embodiment, the thickener is present in an amount of from 0.5% to 3% by weight.

The diaper rash composition may comprise further optional ingredients in addition to those discussed above. For example, any of the following is a suitable additive for the diaper rash composition: fragrance, preservative, pH adjuster, pigment, dye, anti-microbial, emollient, moisturizer, and combinations thereof. The diaper rash composition may potentially contain zero, one, or a plurality of any of the listed additives.

### EXAMPLES

The following are examples of diaper rash compositions that may be made in accordance with the present disclosure.

### Example 1

**Table 1. Diaper Rash Composition 1**

| **Trade Name** | **Ingredient** | **% wt** | **Supplier** |
|---|---|---|---|
| DOW CORNING 245 | cyclopentasiloxane | 33.0 | Dow Corning Corp. |
| DOW CORNING 9040 | cyclopentasiloxane and dimethicone crosspolymer | 52.0 | Dow Corning Corp. |
| ZCOTE HP1 | zinc oxide | 15.0 | BASF |
| **Total** | | **100.0** | |

As shown in the above embodiment, the diaper rash composition of the present disclosure may contain a single volatile silicone, silicone elastomer, and diaper rash agent. The volatile silicone in Table 1 is cyclopentasiloxane, sold under the name DOW CORNING 245. The silicone elastomer is a blend of silicone elastomer and cyclomethicone, sold by the Dow Corning Corp. under the name DOW CORNING 9040. The weight percentage shown in Example 1 gives the percentage of silicone elastomer blend. Zinc oxide (obtained from BASF) serves as the diaper rash agent in Example 1.

To make the diaper rash composition of Example 1. The three components listed in the table were combined and mixed at room temperature until the composition had reached a homogenous state.

### Example 2

**Table 2. Diaper Rash Composition 2**

| **Trade Name** | **Ingredient** | **% wt** | **Supplier** |
|---|---|---|---|
| DOW CORNING 245 | cyclopentasiloxane | 24.0 | Dow Corning Corp. |
| DOW CORNING 9040 | cyclopentasiloxane and dimethicone crosspolymer | 56.0 | Dow Corning Corp. |
| DOW CORNING 9011 | cyclopentasiloxane and PEG-12 dimethicone crosspolymer | 10.0 | Dow Corning Corp. |
| ZCOTE HP1 | zinc oxide | 10.0 | BASF |
| **Total** | | **100.0** | |

The diaper rash composition shown in the above embodiment comprises the same components as Example 1 with one addition. In the given embodiment, both DOW CORNING 9040 and DOW CORNING 9011 are present in the composition as silicone elastomers. The 9011 formulation is a blend of PEG-12 dimethicone crosspolymer and cyclomethicone. The table shows that commercially available silicone elastomer blends are present in an amount of 66.0% by weight of the total composition.

The four ingredients in Table 2 were combined at room temperature in the given amounts and mixed until homogeneous to create a diaper rash composition.

### Example 3

**Table 3. Diaper Rash Composition 3**

| **Trade Name** | **Ingredient** | **% wt** | **Supplier** |
|---|---|---|---|
| DOW CORNING 245 | cyclopentasiloxane | 13.0 | Dow Corning Corp. |
| DOW CORNING 9040 | cyclopentasiloxane and dimethicone crosspolymer | 64.0 | Dow Corning Corp. |
| ZCOTE HP1 | zinc oxide | 10.0 | BASF |
| TEGOSOFT M | Isopropyl myristate | 13.0 | Degussa |
| **Total** | | **100.00** | |

An optional ingredient that may be present in the diaper rash composition is a carrier, which is isopropyl myristate in the given example. Isopropyl myristate is sold by Degussa under the name TEGOSOFT M. The volatile silicone, silicone elastomer, and diaper rash agent are present in the composition, as well.

In order to create the diaper rash composition, the four ingredients listed were combined and mixed until the composition reached a homogenous state.

### Example 4

**Table 4. Diaper Rash Composition 4**

| **Trade Name** | **Ingredient** | **% wt** | **Supplier** |
|---|---|---|---|
| DOW CORNING 245 | cyclopentasiloxane | 13.0 | Dow Corning Corp. |
| DOW CORNING 9040 | cyclopentasiloxane and dimethicone crosspolymer | 63.0 | Dow Corning Corp. |
| ZCOTE HP1 | zinc oxide | 10.0 | BASF |
| TEGASOFT M | Isopropyl myristate | 13.0 | Degussa |
| LAPONITE | magnesium silicate | 1.0 | Rockwood Additives Ltd. Corp. |
| **Total** | | **100.00** | |

As shown above, the diaper rash composition may comprise a volatile silicone, a silicone elastomer, a diaper rash agent, a carrier, and a thickener. In the given embodiment, the carrier is isopropyl myristate and the thickener is magnesium silicate. Magnesium silicate is sold under the name LAPONITE and is distributed by Rockwood Additives Ltd. Corp.

In order to make the diaper rash composition of the present example, the components given in Table 4 were combined. Then, the composition was mixed until it reached a homogenous state.

### Example 5

**Table 5. Diaper Rash Composition 5**

| **Trade Name** | **Ingredient** | **% wt** | **Supplier** |
|---|---|---|---|
| DOW CORNING 245 | cyclopentasiloxane | 11.62 | Dow Corning Corp. |
| DOW CORNING 9040 | cyclopentasiloxane and dimethicone crosspolymer | 32.25 | Dow Corning Corp. Dow Corning Corp. |
| ZCOTE HP1 | zinc oxide | 7.74 | BASF |
| VERSAGEL MD 1600 | isodecane with ethylene/propylene/styrene and butylene/ethylene/styrene block copolymers | 38.71 | Penreco |
| SNOW WHITE | petrolatum | 9.68 | Penreco |
| **Total** | | **100.00** | |

In diaper rash composition 5, the volatile silicone, the silicone elastomer, and the diaper rash agent were present as in the other compositions. Both isododecane with ethylene/propylene/styrene and butylene/ethylene/styrene block copolymers, sold under the name VERSAGEL MD 1600, and petrolatum served as carriers in the composition.

The components listed in Table 5 were combined and mixed until the composition became homogenous.

## Claims

1. A diaper rash composition comprising:
a volatile silicone present in the diaper rash composition in an amount greater than 10% by weight;
a silicone elastomer comprising a crosslinked reaction product of a polysiloxane containing a ≡Si-H group and an alpha, omega-diene;
a diaper rash agent; and
wherein the diaper rash composition is substantially non-aqueous containing less than 1% by weight water, contains no emulsifiers and is a semi-solid.

2. A diaper rash composition as defined in claim 1, wherein the polysiloxane containing a ≡Si-H group comprises
R₃SiO(R'₂SiO)ₐ(R"HSiO)_{b}SiR₃,
HR₂SiO(R'₂SiO)_{c}SiR₂H,
HR₂SiO(R'₂SiO)ₐ(R"HSiO)_{b}SiR₂H,
or mixtures thereof, wherein R, R', and R" are alkyl groups with 1 to 6 carbon atoms, a is 0 to 250, b is 1 to 250, and c is 0 to 250; and
wherein the alpha, omega-diene comprises
CH₂=CH(CH₂)ₓCH=CH₂
wherein x is 1 to 20.

3. A diaper rash composition as defined in claim 1 or 2, wherein the volatile silicone comprises cyclomethicone, dimethicone, or mixtures thereof.

4. A diaper rash composition as defined in claim 1, 2 or 3, wherein the volatile silicone is present in an amount of greater than 25% by weight.

5. A diaper rash composition as defined in claim 1, 2, 3 or 4, wherein the silicone elastomer viscosity is greater than 200 Pa s (200,000 cP).

6. A diaper rash composition as defined in claim 1, 2, 3, 4 or 5, wherein the diaper rash agent comprises a zinc oxide, a nonvolatile dimethicone, allantoin, cod liver oil, colloidal oatmeal, kaolin, lanolin, petrolatum, topical starch, or combinations thereof.

7. A diaper rash composition as defined in claim 1, 2, 3, 4, 5 or 6, wherein the composition further comprises a non-silicone elastomer.

8. A diaper rash composition as defined in claim 7, wherein the non-silicone elastomer comprises a styrenic block copolymer.

9. A diaper rash composition as defined in any of the preceding claims, wherein the composition further comprises a carrier in an amount of at least 5% by weight.

10. A diaper rash composition as defined in claim 9, wherein the carrier comprises isopropyl myristate, isododecane, or mixtures thereof.

11. A diaper rash composition as defined in claim 9, wherein the carrier comprises a light hydrocarbon oil, a vegetable oil, a natural oil, a hydrogenated vegetable oil, a fatty ester, a fatty acid, a fatty alcohol, or combinations thereof.

12. A diaper rash composition as defined in any of the preceding claims, wherein the composition further comprises a thickener.

13. A diaper rash composition as defined in claim 12, wherein the thickener comprises a clay.

14. A diaper rash composition as defined in any of the preceding claims, wherein the diaper rash agent is present in an amount from 10% to 40% by weight.

15. A diaper rash composition as defined in any of the preceding claims, wherein the silicone elastomer is present in the composition in an amount from 3% to 10% by weight.

16. A diaper rash composition as defined in any of the preceding claims wherein the diaper rash composition contains less than 0.5% water by weight.

17. A diaper rash composition as defined in any of the preceding claims wherein the diaper rash composition contains no water.

## Patentansprüche

1. Windelausschlagzusammensetzung, umfassend:
flüchtiges Silikon, welches in der Windelausschlagzusammensetzung in einer Menge von mehr als 10 Gewichtsprozent vorhanden ist;
ein Silikonelastomer, welches ein vernetztes Reaktionsprodukt eines Polysiloxans enthaltend eine ≡Si-H Gruppe und eines alpha,omega-Diens ist;
ein Windelausschlagsmittel; und
wobei die Windelausschlagzusammensetzung im Wesentlichen nicht wässrig, weniger als 1 Gewichtsprozent Wasser enthaltend ist, keine Emulgatoren enthält und halbfest ist.

2. Windelausschlagzusammensetzung gemäß Anspruch 1, wobei das Polysiloxan enthaltend eine ≡Si-H Gruppe
R₃SiO(R'₂SiO)ₐ(R"HSiO)_{b}SiR₃,
HR₂SiO(R'₂SiO)_{c}SiR₂H,
HR₂SiO(R'₂SiO)ₐ(R"HSiO)_{b}SiR₂H,
oder Mischungen davon umfasst, wobei R, R', and R" Alkylgruppen mit 1 bis 6 Kohlenstoffatomen sind, a 0 bis 250 ist, b 1 bis 250 ist und c 0 bis 250 ist; und
wobei das alpha,omega Dien
CH₂=CH(CH₂)ₓCH=CH₂
umfasst, wobei x 1 bis 20 ist.

3. Windelausschlagzusammensetzung gemäß Anspruch 1 oder 2, wobei das flüchtige Silikon Cyclomethicon, Dimethicon oder Mischungen davon umfasst.

4. Windelausschlagzusammensetzung gemäß Anspruch 1, 2 oder 3, wobei das flüchtige Silikon in einer Menge von mehr als 25 Gewichtsprozent vorhanden ist.

5. Windelausschlagzusammensetzung gemäß Anspruch 1, 2, 3 oder 4, wobei die Viskosität des Silikonelastomers größer ist als 200 Pa s (200.000 cP).

6. Windelausschlagzusammensetzung gemäß Anspruch 1, 2, 3, 4 oder 5, wobei die Windelausschlagszusammensetzung ein Zinkoxid, ein nicht flüchtiges Dimethicon, Allantoin, Lebertran, kolloides Hafermehl, Kaolin, Lanolin, Petrolatum, topische Stärke oder Kombinationen davon umfasst.

7. Windelausschlagzusammensetzung gemäß Anspruch 1, 2, 3, 4, 5 oder 6, wobei die Zusammensetzung des Weiteren ein Nichtsilikonelastomer umfasst.

8. Windelausschlagzusammensetzung gemäß Anspruch 7, wobei das Nichtsilikonelastomer ein Styrol-Block-Copolymer umfasst.

9. Windelausschlagzusammensetzung gemäß einem der vorherigen Ansprüche, wobei die Zusammensetzung des Weiteren einen Träger in einer Menge von mindestens 5 Gewichtsprozent umfasst.

10. Windelausschlagzusammensetzung gemäß Anspruch 9, wobei der Träger Isopropylmyristat, Isododecan oder Mischungen davon umfasst.

11. Windelausschlagzusammensetzung gemäß Anspruch 9, wobei der Träger ein leichtes Kohlenwasserstofföl, ein pflanzliches Öl, ein natürliches Öl, ein hydriertes pflanzliches Öl, einen Fettsäureester, eine Fettsäure, einen Fettalkohol oder Kombinationen davon umfasst.

12. Windelausschlagzusammensetzung gemäß einem der vorherigen Ansprüche, wobei die Zusammensetzung des Weiteren ein Verdickungsmittel umfasst.

13. Windelausschlagzusammensetzung gemäß Anspruch 12, wobei das Verdickungsmittel Tonerde umfasst.

14. Windelausschlagzusammensetzung gemäß einem der vorherigen Ansprüche, wobei das Windelausschlagsmittel in einer Menge von 10 Gewichtsprozent bis 40 Gewichtsprozent vorhanden ist.

15. Windelausschlagzusammensetzung gemäß einem der vorherigen Ansprüche, wobei das Silikonelastomer in der Zusammensetzung in einer Menge von 3 Gewichtprozent bis 10 Gewichtsprozent vorhanden ist.

16. Windelausschlagzusammensetzung gemäß einem der vorherigen Ansprüche, wobei die Windelausschlagszusammensetzung weniger als 0,5 % Gewichtsprozent Wasser enthält.

17. Windelausschlagzusammensetzung gemäß einem der vorherigen Ansprüche, wobei die Windelausschlagszusammensetzung kein Wasser enthält.

## Revendications

1. Composition anti-érythème fessier comprenant :
un silicone volatil présent dans la composition anti-érythème fessier en une quantité supérieure à 10 % en poids ;
un élastomère de silicone comprenant un produit de réaction réticulé d'un polysiloxane contenant un groupe ≡Si-H et d'un alpha,oméga-diène ;
un agent anti-érythème fessier;
ladite composition anti-érythème fessier étant sensiblement non-aqueuse et contenant moins de 1 % en poids d'eau, ne contenant pas d'émulsifiants et étant semi-solide.

2. Composition anti-érythème fessier telle que définie dans la revendication 1, dans laquelle le polysiloxane contenant un groupe ≡Si-H comprend
R₃SiO(R'₂SiO)ₐ(R"HSiO)_{b}SiR₃,
HR₂SiO(R'₂SiO) _{c}SiR₂H,
HR₂SiO(R'₂SiO)ₐ(R"HSiO)_{b}SiR₂H
ou leurs mélanges, où R, R' et R" sont des groupes alkyle ayant de 1 à 6 atomes de carbone, a représente de 0 à 250, b représente de 1 à 250 et c représente de 0 à 250 ; et
dans laquelle l'alpha,oméga-diène comprend
CH₂=CH (CH₂) ₓCH=CH₂
où x représente de 1 à 20.

3. Composition anti-érythème fessier telle que définie dans la revendication 1 ou 2, dans laquelle le silicone volatil comprend le cyclométhicone, le diméthicone, ou leurs mélanges.

4. Composition anti-érythème fessier telle que définie dans la revendication 1, 2 ou 3, dans laquelle le silicone volatil est présent en une quantité supérieure à 25 % en poids.

5. Composition anti-érythème fessier telle que définie dans la revendication 1, 2, 3 ou 4, dans laquelle la viscosité de l'élastomère de silicone est supérieure à 200 Pa.s (200 000 cP).

6. Composition anti-érythème fessier telle que définie dans la revendication 1, 2, 3, 4 ou 5, dans laquelle l'agent anti-érythème fessier comprend un oxyde de zinc, un diméthicone non-volatil, de l'allantoïne, de l'huile de foie de morue, de l'avoine colloïdale, du kaolin, de la lanoline, de la vaseline, de l'amidon topique ou des combinaisons de ceux-ci.

7. Composition anti-érythème fessier telle que définie dans la revendication 1, 2, 3, 4, 5 ou 6, ladite composition comprenant, en outre, un élastomère non-silicone.

8. Composition anti-érythème fessier telle que définie dans la revendication 7, dans laquelle l'élastomère non-silicone comprend un copolymère séquencé styrènique.

9. Composition anti-érythème fessier telle que définie dans l'une quelconque des revendications précédentes, ladite composition comprenant, en outre, un support en une quantité d'au moins 5 % en poids.

10. Composition anti-érythème fessier telle que définie dans la revendication 9, dans laquelle le support comprend le myristate d'isopropyle, l'isododécane, ou leurs mélanges.

11. Composition anti-érythème fessier telle que définie dans la revendication 9, dans lequel le support comprend une huile d'hydrocarbure léger, une huile végétale, une huile naturelle, une huile végétale hydrogénée, un ester gras, un acide gras, un alcool gras ou une combinaison de ceux-ci.

12. Composition anti-érythème fessier telle que définie dans l'une quelconque des revendications précédentes, ladite composition comprenant, en outre, un épaississant.

13. Composition anti-érythème fessier telle que définie dans la revendication 12, dans laquelle l'épaississant comprend une argile.

14. Composition anti-érythème fessier telle que définie dans l'une quelconque des revendications précédentes, dans laquelle l'agent anti-érythème fessier est présent en une quantité allant de 10 % à 40 % en poids.

15. Composition anti-érythème fessier telle que définie dans l'une quelconque des revendications précédentes, dans laquelle l'élastomère de silicone est présent dans la composition en une quantité allant de 3 % à 10 % en poids.

16. Composition anti-érythème fessier telle que définie dans l'une quelconque des revendications précédentes, ladite composition anti-érythème fessier contenant moins de 0,5 % en poids d'eau.

17. Composition anti-érythème fessier telle que définie dans l'une quelconque des revendications précédentes, ladite composition anti-érythème fessier ne renfermant pas d'eau.
